# FASCICULE DE BREVET EUROPEEN

(11) **EP 2 806 841 B1**
(45) Date de publication et mention de la délivrance du brevet: **06.01.2016**
(21) Numéro de dépôt: 13710559.9
(22) Date de dépôt: 24.01.2013
(51) Int. Cl.: A61F 11/00, A61F 13/38, A61B 10/02

(54) **PROCÉDÉ DE FABRICATION D'UN INSTRUMENT DE CURETAGE D'UN CONDUIT CORPOREL ET INSTRUMENT AINSI OBTENU**
VERFAHREN ZUR HERSTELLUNG EINES INSTRUMENT ZUR KÜRETTAGE EINES KÖRPERKANALS UND RESULTIERENDES INSTRUMENT
METHOD FOR PRODUCING AN INSTRUMENT FOR THE CURETTAGE OF A BODILY CONDUIT AND RESULTING INSTRUMENT

(30) Priorité: 24.01.2012 FR 1250685
(43) Date de publication de la demande: 03.12.2014
(73) Titulaire: Dinamic Emballages, 68660 Rombach Le Franc (FR)
(72) Inventeur: SITTLER, Jean-Pierre, 67230 Herbsheim (FR)
(74) Mandataire: Merckling, Norbert
(86) Numéro de dépôt international: PCT/IB2013/050620
(87) Numéro de publication internationale: WO 2013/111089

(56) Documents cités:
- EP-A1- 0 875 221
- EP-A1- 1 053 727
- WO-A1-91/16855
- WO-A1-02/080776
- FR-A1- 2 794 355
- FR-A1- 2 830 432
- US-A- 3 312 215

## Description

La présente invention se rapporte au domaine technique général des instruments de nettoyage ou de curetage manuels.

La présente invention concerne plus particulièrement un procédé de fabrication d'un instrument de curetage ou de prélèvement manuel, du type bâtonnet, dans un conduit corporel naturel ou artificiel.

On connait l'instrument manuel classique à extrémités en ouate, reliées par une tige dont l'ensemble est appelé communément coton-tige.

Cet instrument a été perfectionné par des extrémités en forme de bulbe creux dont la surface latérale est constituée d'une succession de lamelles réunies par leurs extrémités pour former le dôme arrondi du bulbe.

Ces lamelles doivent être réalisées en matière plastique pour conférer au bulbe le caractère de souplesse nécessaire à sa déformation en vrille suite à un mouvement de pivotement de l'instrument sur lui-même lorsqu'une de ses extrémités se trouve à l'intérieur d'un conduit naturel corporel, par exemple le conduit auditif dans le cas d'un cure-oreille.

Un exemple d'un tel instrument de ce type est décrit dans le brevet français FR 2 760 633 au nom de Monsieur Jean-Pierre SITTLER.

Pour réaliser le bulbe par les techniques de moulage par injection, on le moule en deux demi parties articulées entre elles par une charnière connue selon la description du brevet français FR 2 794 355 et du brevet européen EP 0875221. Le brevet européen EP 1053727 divulgue les caractéristiques du préambule de la revendication 1.

La forme de réalisation ainsi décrite concerne un bulbe en deux demi parties articulées entre elles par charnière.

Selon le descriptif de ces brevets, l'une de ces demi parties de bulbe, présente une extrémité conformée en un bout de tige venant s'engager à force après rabattement dans un espace creux en fente plus étroite, prévu à l'extrémité du bâtonnet.

Selon cette technique, l'extrémité de l'autre de mi-partie du bulbe vient se confondre avec l'extrémité du bâtonnet comme visible sur la figure 1 du brevet français cité ci-dessus.

Ceci oblige, pour rester dans un procédé de fabrication simple, à réaliser la tige creuse et le bulbe dans la même matière que celle de la tige.

Ce type de formes techniques complémentaires d'assemblage des deux demi-bulbes avec la tige, ne donne pas satisfaction pour différentes raisons.

Celles-ci sont liées à la résistance de la matière plastique, à la solidité de la liaison d'assemblage du bulbe à l'extrémité de la tige et à la durée de l'opération d'assemblage, mais aussi à la difficulté de l'insertion par pression de l'extrémité en bout de tige du bulbe dans la fente d'extrémité de la tige.

De plus, la tige et sa liaison d'extrémité avec le bulbe doivent présenter une solidité suffisante pour le travail demandé.

Toutes ces raisons n'ont permis ni de fabriquer l'instrument cure-oreilles en quantités industrielles telles que le demande le marché, ni de lui conférer les qualités techniques nécessaires à une utilisation efficace.

Or, s'agissant d'un article jetable, la production de masse de celui-ci est une condition essentielle à son utilisation et par conséquent au développement commercial de celui-ci.

Par ailleurs, la liaison d'extrémité de la tige avec le bulbe doit être suffisamment résistante pour éviter une rupture à ce niveau, voire une simple déchirure qui ne peut assurer le maintien de l'intégralité du bulbe lors du mouvement de curetage et lors du retrait du conduit. De plus, le bulbe, lequel est appelé à venir en contact avec des parties sensibles du corps humain, doit être réalisé dans une matière souple, tendre et d'un contact doux.

La présente invention a pour but de remédier à ces inconvénients et à produire cet instrument en grande série par l'intermédiaire d'un procédé de fabrication simple et peu coûteux, présentant notamment un nombre réduit d'étapes de mise en oeuvre.

Un objectif de l'invention vise également à fournir un instrument de nettoyage amélioré dont les caractéristiques techniques sont satisfaisantes notamment du point de vue solidité mécanique et ceci à un coût unitaire modique.

Un autre objectif de l'invention vise à fournir un instrument de nettoyage amélioré présentant des propriétés de raclage et de curetage, optimisées.

Les objectifs assignés à l'invention sont atteints à l'aide d'un procédé de fabrication d'un instrument de nettoyage, de prélèvement ou autre d'un ou dans un conduit corporel naturel ou artificiel d'un corps humain ou animal du type à tige dont chaque extrémité comporte un bulbe flexible d'extrémité formé d'une succession de lamelles, chaque bulbe flexible étant formé par la superposition de deux demi-bulbes articulés entre eux par une charnière et assemblés par recouvrement et immobilisation de leurs extrémités par lesquelles elles sont reliées à l'extrémité correspondante de la tige, caractérisé en ce que l'on :
- forme par injection d'une première matière dans un moule une structure de base présentant deux groupes d'extrémité de deux demi-bulbes à plat portés chacun dans une partie de moule à cale pivotante,
- forme par injection de cette première matière dans un canal de liaison, une liaison entre les deux groupes d'extrémité de manière à constituer entièrement la structure de base, en rabattant les deux cales pivotantes portant les deux demi-bulbes de chaque groupe d'extrémité,
- engage les extrémités d'assemblage de chaque demi-bulbe correspondant l'une dans l'autre pour fermer les deux demi-bulbes de chaque bulbe et constituer ainsi des bulbes d'extrémité entiers,
- modifie le moule pour agrandir le canal de liaison et constituer des cavités ou chambres périphériques au niveau des jonctions entre les extrémités de la tige et celle des bulbes d'extrémité, ainsi que des espaces pour la formation d'une surépaisseur périphérique au niveau de chaque extrémité,
- injecte une deuxième matière pour former la tige et par extension sur et au-delà des extrémités de la tige et sur les extrémités assemblées des demi-bulbes de chaque bulbe d'extrémité par la deuxième matière une surépaisseur de solidarisation formant un bourrelet ou une bague de jonction et de solidarisation destinée à renforcer la jonction entre l'extrémité de la tige et chaque bulbe d'extrémité.

A titre d'exemple, la liaison entre les deux bulbes d'extrémité est de préférence une liaison filaire.

Selon un exemple de mise en oeuvre du procédé conforme à l'invention, on augmente la section du canal avant l'injection de la deuxième matière.

Selon un exemple de mise en oeuvre du procédé conforme à l'invention, on forme la tige par injection autour de la liaison de la structure de base reliant les deux bulbes d'extrémité.

A titre d'exemple, la première matière est préférentiellement un polyéthylène et la deuxième matière est préférentiellement un polypropylène.

Les objectifs assignés à l'invention sont atteints également à l'aide d'un instrument de nettoyage, de prélèvement obtenu par le procédé présenté ci-dessus, comportant une tige et au moins un moyen de curetage solidaire de l'une des extrémités de la tige, ledit moyen de curetage étant constitué d'une pluralité de lamelles individualisées, lesquelles sont reliées entre elles au niveau d'une de leurs extrémités et solidarisées par leur autre extrémité à l'extrémité de la tige, de manière à constituer à cette extrémité de la tige une forme bombée susceptible de déformation, caractérisé en ce qu'au moins quelques unes des lamelles présentent en saillie sur leur face extérieure délimitant la forme bombée, des brins flexibles.

Selon un exemple de réalisation de l'instrument de nettoyage conforme à l'invention, les brins flexibles sont réalisés d'une seule pièce avec les lamelles.

Selon un exemple de réalisation de l'instrument de nettoyage conforme à l'invention, les brins flexibles présentent de préférence un diamètre inférieur à 0,3 mm.

Selon un exemple de réalisation de l'instrument de nettoyage conforme à l'invention, les brins flexibles présentent de préférence une longueur comprise entre 1,5 et 3 mm.

Selon un exemple de réalisation de l'instrument de nettoyage conforme à l'invention, l'espacement entre les brins flexibles est compris de préférence entre 1 et 3 mm.

Selon un exemple de réalisation de l'instrument de nettoyage conforme à l'invention, la tige comporte à une extrémité, un premier moyen de curetage dont les lamelles présentent des brins flexibles et à une autre extrémité un second moyen de curetage dont les lamelles sont dépourvues de brins flexibles.

Selon un autre exemple de réalisation de l'instrument de nettoyage conforme à l'invention, la tige comporte à chacune de ses extrémités, un moyen de curetage dont les lamelles présentent des brins flexibles.

D'autres caractéristiques et avantages de l'invention apparaîtront dans la description qui suit, donnée à titre d'exemple non limitatif et accompagnée des dessins dans lesquels :

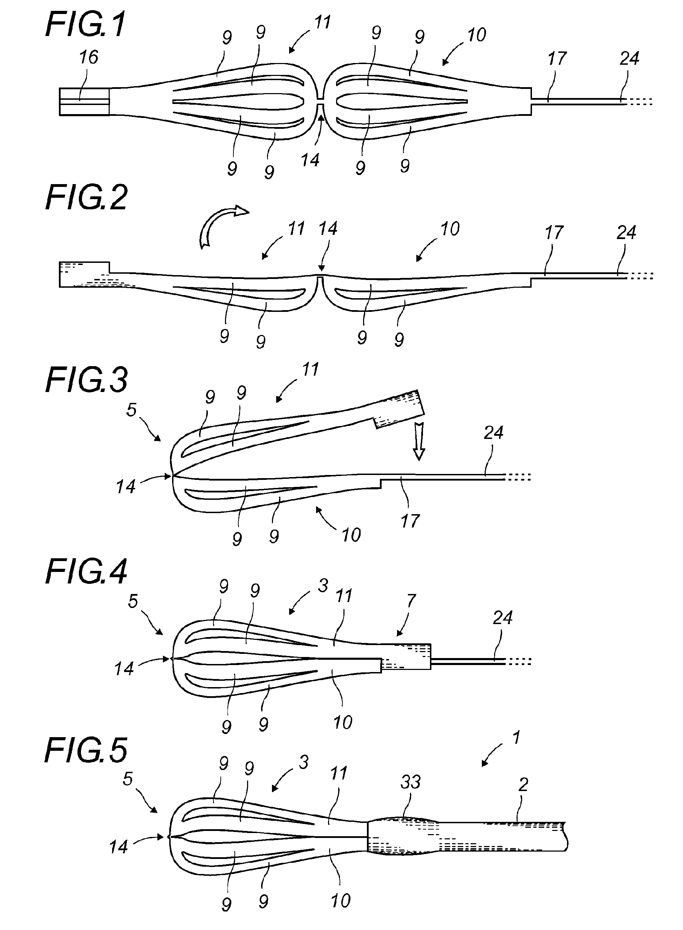

- les figures 1 à 5 sont des vues schématiques de profil d'une extrémité en bulbe montrant avec:
   . la figure 1, une vue en plan d'un bulbe ouvert,
   . la figure 2, une vue de profil d'un bulbe ouvert en deux demi parties avant rabattement,
   . la figure 3, une vue de profil du bulbe juste avant assemblage des deux demi-bulbes,
   . la figure 4, une vue de profil du bulbe après assemblage des extrémités de ses deux demi-bulbes, le bulbe étant prolongé par une liaison de matière,
   . la figure 5, une vue de profil de l'extrémité terminée avec l'extrémité du bulbe solidarisée à l'extrémité de la tige du bâtonnet,

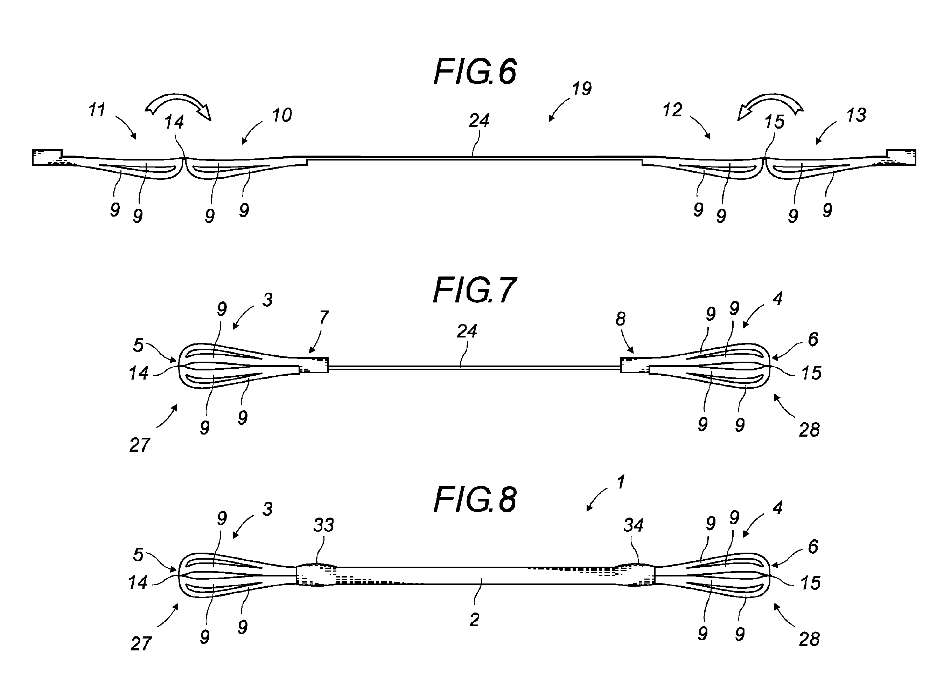

- les figures 6 à 8 sont des vues du bâtonnet lors des trois étapes caractéristiques du procédé de fabrication dont :
   . la figure 6, une vue de profil de la forme de base avec les bulbes d'extrémité ouverts reliés par une liaison de matière,
   . la figure 7, une vue de profil de la forme de base avec les bulbes rabattus,
   . la figure 8, une vue de profil du bâtonnet terminé prêt à son utilisation,
- les figures 9 à 11 sont des vues schématiques des trois stades caractéristiques du procédé de fabrication illustrant par :
   . la figure 9, la formation de la structure de base par injection avec constitution des deux demi-bulbes reliés par une liaison de matière,
   . la figure 10, le rabattement des demi-bulbes d'extrémité,
   . la figure 11, la formation de la tige et de l'enserrement des extrémités par injection,
   - la figure 12 est une vue en perspective de l'extrémité d'un instrument de nettoyage conforme à l'invention, comportant un bâtonnet pourvue d'un bulbe flexible avec des brins sur chacune de ses lamelles,
   - la figure 13 est une vue en perspective d'un instrument de nettoyage conforme à l'invention comportant un bâtonnet complet mixte, présentant une extrémité à bulbe flexible lisse et une extrémité opposée à bulbe flexible avec brins,
   - et la figure 14 est une vue en coupe du moule de production de bâtonnets à bulbes avec un seul bulbe à brins d'un instrument de nettoyage au niveau d'une empreinte.

Les inconvénients et les défauts de l'instrument de l'art antérieur sont remédiés par le procédé de fabrication selon la présente invention et l'instrument en résultant.

Le produit résultant du procédé de fabrication selon l'invention est un bâtonnet 1 à usage de nettoyage des conduits naturels ou artificiels du corps humain ou animal et autres applications possibles.

Il se compose d'une tige 2, terminée à chacune de ses extrémités par un bulbe 3 et 4 présentant chacun un dôme 5 ou 6. Chaque dôme est relié à une extrémité de fermeture 7 ou 8, par une pluralité de lamelles telles que 9 formées dans une matière souple. Cette matière souple présentera de préférence des caractéristiques lui conférant un contact doux avec les parois du conduit à nettoyer tout en assurant une aptitude à un raclage de nettoyage. Les lamelles 9 sont reliées entre elles par les extrémités de fermeture 7 et 8 des bulbes.

Selon les inventions antérieures, ces bulbes d'extrémité 3 et 4 de la tige 2 sont constitués par deux demi-bulbes 10 et 11 et 12 et 13 respectivement intérieurs et extérieurs, reliés chacun entre eux par une articulation à charnière 14 ou 15 permettant de les faire pivoter l'un sur l'autre lors d'un mouvement de rabattement. Les demi-bulbes 10 à 13 sont immobilisés l'un sur l'autre par un engagement ou un encastrement de formes complémentaires par exemple une forme réceptrice linéaire à gorge telle que 16 par exemple en U inversé venant chevaucher puis s'appuyer sur ou enserrer, une contre-forme linéaire complémentaire 17 en relief comme représenté sur les dessins. Les extrémités de fermeture, 7 et 8 sont donc formées de deux extrémités complémentaires venant s'encastrer à immobilisation l'une dans l'autre. Pour les deux parties de ces extrémités de fermeture, bien d'autres formes techniques complémentaires peuvent être imaginées. Il suffit de respecter la fonction générale qui est d'arriver de façon simple à un blocage minimal ou temporaire en fermeture entre chaque extrémité de chaque demi-bulbe.

Selon la présente invention, ce type de bâtonnet 1 peut être fabriqué en grande série par le procédé de moulage qui sera décrit ci-après permettant d'obtenir une liaison résistante entre la tige et les extrémités en bulbes formant un bâtonnet de nettoyage, de curage ou de curetage et de prélèvement.

On forme d'abord par injection d'une première matière dans un moule d'injection 18 une structure de base 19 constituée des deux groupes de demi-bulbes en position complètement ouverte dans laquelle ils se trouvent à plat reliés entre eux par l'articulation de pivotement à charnière 14 ou 15. Le moule 18 est ainsi conformé pour que l'injection de la première matière s'effectue en partie centrale entre les deux demi bulbes intérieurs de chaque groupe. Ces derniers sont situés chacun à l'extrémité d'un canal de liaison 20 par lequel est injecté la première matière délivrée par une buse 21. Les extrémités de ce canal de liaison 20 débouchent chacune dans une cavité de moule 22 et 23 présentant chacune les contre-formes des deux demi-bulbes s'étendant à plat l'un à la suite de l'autre et reliés entre eux par la charnière 14 ou 15 dans la même matière souple. L'injection de la première matière, qui peut être un polyéthylène, délivrée par la buse 21 le long du canal de liaison 20 arrive aux cavités 22 et 23 pour former les demi-bulbes de chaque extrémité. Elle remplit ensuite le canal de liaison 20 de manière à obtenir après l'injection, une structure de base constituée des deux groupes d'extrémité des deux demi-bulbes reliés entre eux par une liaison 24 de la première matière. Cette liaison 24 de la première matière peut être quelconque par exemple du type filaire, en principe de faible épaisseur, par exemple égale à celle de la section du canal de liaison 20 de manière à garantir l'espacement entre les deux futurs bulbes d'extrémité. Elle est destinée à former un support primaire pour la tige 2 du bâtonnet.

La première matière est celle formant les bulbes d'extrémité c'est-à-dire la matière souple et tendre à la fois destinée à réaliser le contact doux avec la paroi du conduit à nettoyer à l'aide de l'instrument selon l'invention.

Chaque cavité formant chaque demi-bulbe d'extrémité est supportée par une cale pivotante 25 et 26 permettant à chaque fois le rabattement du demi-bulbe d'extrémité 11 ou 13 sur le demi bulbe intérieur 10 ou 12. Ce mouvement s'effectue autour de la charnière 14 ou 15, qui sert d'articulation de pivotement entre deux demi bulbes successifs.

Après une durée suffisante suivant l'injection de la première matière, on procède de chaque côté du moule au rabattement des demi-bulbes d'extrémité sur les demi-bulbes correspondants à l'aide des cales pivotantes 25 et 26 dont est équipé le moule 18. A la fin de ce mouvement de rabattement, chaque bulbe est entièrement constitué c'est-à-dire qu'il se trouve formé par la superposition de deux demi-bulbes correspondants.

A cette étape du procédé, les deux demi-bulbes d'un même bulbe d'extrémité sont assemblés par leurs extrémités par encastrement plus ou moins ajusté de formes techniques, par exemple par l'intermédiaire des formes techniques d'extrémité 16 et 17. Cet ajustement peut être plus ou moins serré selon les besoins. Il s'agit de l'engagement de formes complémentaires, notamment les formes d'extrémité, dont un exemple a été décrit ci-dessus au niveau des extrémités de fermeture 7 et 8.

Lorsque chaque demi-bulbe extérieur 11 ou 13 est rabattu sur le demi-bulbe intérieur correspondant 10 ou 12, les bords périphériques se recouvrent pour former un bulbe complet homogène 27 et 28 qui présente déjà une bonne cohésion après immobilisation suite à l'engagement des formes techniques complémentaires d'extrémité avec plus ou moins de force de pression.

La deuxième partie du procédé se déroule de la façon suivante.

La structure de base 19 avec les bulbes 27 et 28 formés et restant en place dans la configuration où les cales pivotantes sont rabattues, on procède à l'injection d'une deuxième matière destinée à former la tige 2 et un bourrelet d'enserrement et de solidarisation des extrémités des bulbes et celles de la tige 2.

A cet effet, on déplace le moule sous une deuxième buse 29 ou l'on translate cette deuxième buse 29 jusqu'au canal de liaison 20. Cette deuxième buse 29 est destinée à délivrer une deuxième matière plastique qui doit être notablement plus résistante que la première matière et compatible avec elle.

L'injection peut s'effectuer également en position centrale de la buse 29.

Pour permettre la réalisation de la tige 2 dont le diamètre est supérieur à la liaison filaire de la structure de base, il y a lieu d'agrandir le canal de liaison 20 qui devient le conduit 30 de tige. Cette opération s'effectue par toute technique connue de moulage par exemple par translation de pièces. Lors de cette modification de géométrie du moule, on crée au niveau de la zone de chaque extrémité de la tige 2 et de celle des bulbes 27 et 28 une cavité ou chambre périphérique 31 et 32 qui a pour but de permettre à la deuxième matière d'entourer entièrement chacune de ces extrémités en formant une surépaisseur périphérique de renforcement en forme de bourrelet, de douille ou de bague de solidarisation 33 et 34 chevauchant les deux extrémités et dont le rôle est de renforcer la résistance de la jonction entre chaque extrémité de la tige 2 et l'extrémité de chaque bulbe 27 et 28. En effet, cette partie est soumise aux efforts de torsion et de traction lors du travail de nettoyage du conduit et doit par conséquent être assez solide.

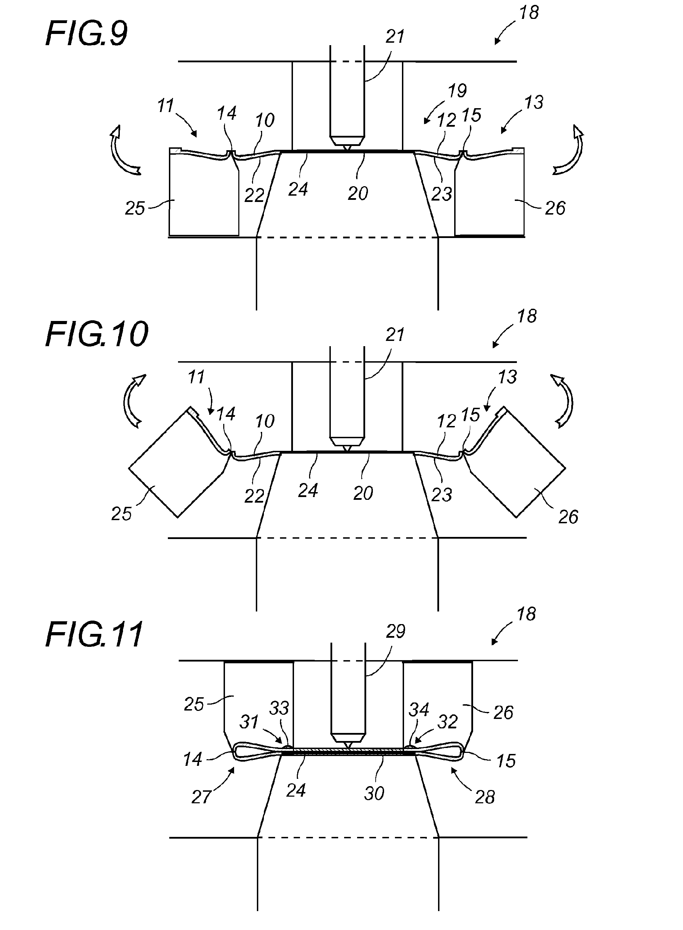

Cette surépaisseur périphérique 33 ou 34 de solidarisation est représentée sur les figures 5 et 10 ainsi que sur la coupe transversale du moule.

La deuxième matière peut être du polypropylène simple ou renforcé de fibres de verre ou de carbone.

A titre d'exemple, la section du canal formant la tige 2, appelée conduit 30 de tige, est majoritairement occupée à chacune de ses extrémités par l'extrémité de chaque bulbe. Il n'existe que peu d'espace au niveau de l'entrée dans les cavités ou chambres périphériques 31 et 32, si bien que l'on réalise une véritable extrusion de la matière à ce niveau qui vient se plaquer sur l'extrémité de raccordement à la tige de chaque bulbe.

Ce procédé à l'avantage de conférer à la liaison entre chaque bulbe 27 et 28 et chaque extrémité de la tige 2, une résistance suffisante par rapport aux efforts physiques développés pour la mise en oeuvre de l'instrument selon l'invention.

On obtient ainsi, un maintien à solidité renforcée suffisante à l'usage pour les extrémités d'une part de la tige et d'autre part du bulbe.

La présente invention se rapporte aussi à l'instrument de nettoyage ou de prélèvement tel qu'obtenu par le procédé décrit ci-dessus.

Une variante perfectionnée de l'invention concerne des bâtonnets dont les lamelles de l'un ou des deux bulbes flexibles d'extrémité comportent des structures 35 fines, saillantes et flexibles par exemple le long de la ligne médiane de chaque lamelle ou d'au moins quelques-unes d'entre elles. Ces structures fines, saillantes et flexibles peuvent être des picots, des tétons, des brins, des poils de brosse, des filaments ou des mini tiges flexibles ou autres éléments analogues.

A titre strictement d'exemple on indique des dimensions préférées à savoir des diamètres de l'ordre de quelques dixièmes de millimètres, par exemple inférieurs à 0,3 mm et des longueurs de quelques millimètres par exemple comprises entre 1,5 mm et 3 mm et des espacements entre ces structures fines de l'ordre de quelques millimètres, par exemple entre 1 mm et 3 mm.

Pour des raisons de simplification dans l'énoncé, on appellera ci-après tous ces structures ou éléments fins et flexibles par le terme unique « brins » 35 qui hérissent longitudinalement chaque lamelle ou seulement quelques-unes d'entre elles par exemple le long d'une ligne médiane pour former un bulbe hérissé 36.

La longueur de ces brins 35 pourra varier de préférence d'une extrémité à l'autre de chaque lamelle de manière à respecter la forme ovoïde du bulbe afin d'entrer progressivement dans le conduit à nettoyer.

La disposition et l'écartement des brins 35 sur chaque lamelle peuvent varier en fonction de l'application et de l'effet recherché. On peut par exemple prévoir plusieurs rangées sur une même lamelle ou sur une demi partie uniquement de celle-ci par exemple sur la partie côté extérieure du bulbe hérissé 36 ou seulement sur une seule lamelle de deux lamelles successives.

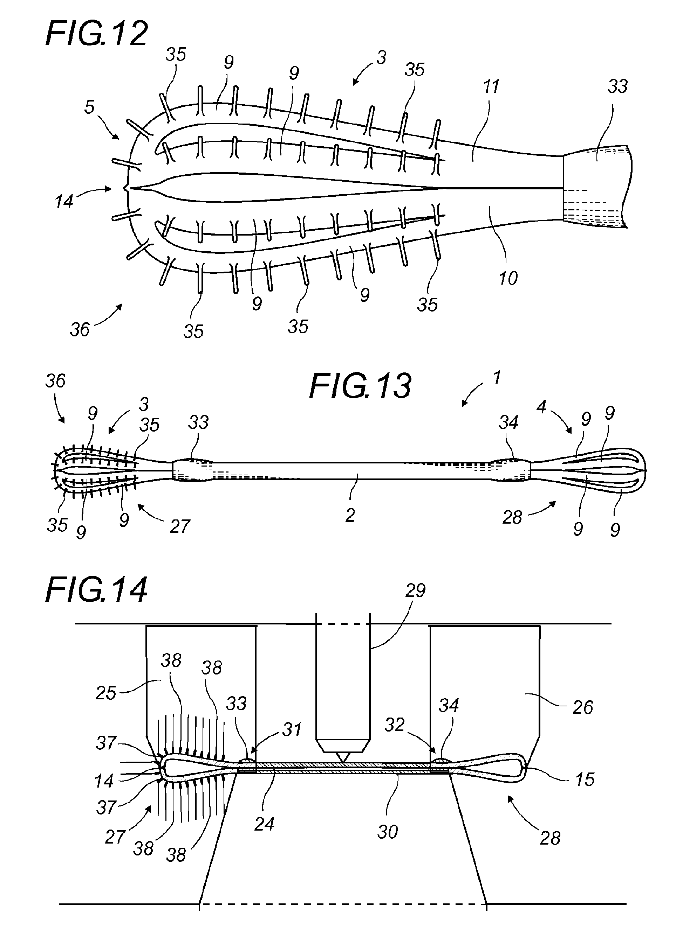

Tel qu'illustré par la figure 14, les brins 35 sont formés lors de la phase de moulage du bâtonnet dans la même matière que celle des lamelles et d'une seule pièce avec celles-ci. On prévoit à cet effet des canaux longitudinaux 37 localement perpendiculaires à la surface en regard des lamelles selon la disposition souhaitées pour ces brins le long des lamelles. Ces canaux sont alimentés en matière par des conduits 38.

Les bâtonnets comportent soit deux bulbes flexibles pourvus de brins 35 soit un bulbe flexible lisse et un bulbe flexible hérissé 36 de brins 35 et un bulbe lisse.

Les bâtonnets présentant chacun un ou deux bulbes d'extrémité dont les lamelles sont pourvues de brins 35 sont des bâtonnets à bulbes hérissés 36. Chaque variante correspond à un usage particulier, celle avec bulbe(s) lisse(s) correspond à un nettoyage en douceur alors que celle avec bulbe(s) à brins permet un nettoyage plus intensif.

Les bâtonnets présentant chacun un ou deux bulbes d'extrémité dont les lamelles sont hérissées de brins 35 sont de produits nouveaux en tant que cure-oreilles ou instruments de nettoyage d'un conduit naturel ou artificiel du corps d'un être vivant ou plus généralement des parois intérieures d'un cavité ou d'un conduit.

On peut à l'aide d'un bâtonnet mixte, c'est-à-dire pourvu d'un côté d'un bulbe sans brins et de l'autre côté d'un bulbe 36 avec brins 35 effectuer un travail de nettoyage en deux temps. Le premier temps est un curage primaire avec le bulbe lisse et le deuxième temps consiste à brosser l'intérieur du conduit pour détacher et entraîner vers la sortie ou emporter avec le bulbe les résidus du curage effectué précédemment ou résultant du doux grattage par les brins.

La présente invention ne se limite pas aux exemples de mise en oeuvre ou de réalisation explicitement décrits ci-dessus. D'autres exemples de mise en oeuvre ou de réalisation font partie de l'invention. Ainsi, une étape du procédé l'invention ou une caractéristique technique de l'invention, peut être remplacée respectivement par une étape équivalente, ou par une caractéristique technique équivalente sans sortir du cadre de l'invention.

## Revendications

1. Procédé de fabrication d'un instrument de nettoyage d'un ou de prélèvement dans un conduit corporel naturel ou artificiel d'un corps humain ou animal du type à tige (2) dont chaque extrémité comporte un bulbe flexible d'extrémité (27) ou (28) formé d'une succession de lamelles (9), chaque bulbe étant formé par la superposition de deux demi-bulbes articulés entre eux par une charnière (14) ou (15) et assemblés par recouvrement et immobilisation de leurs extrémités par lesquelles elles sont reliées à l'extrémité correspondante de la tige (2), dans lequel on :
- forme par injection d'une première matière dans un moule (18) une structure de base (19) présentant deux groupes d'extrémité de deux demi-bulbes (10, 11) ou (12, 13) ouverts à plat portés chacun dans une partie de moule à cale pivotante (25) ou (26),
- forme par injection de cette première matière dans un canal de liaison (20), une liaison (24) entre les deux groupes d'extrémité de manière à constituer entièrement la structure de base (19), en rabattant les deux cales pivotantes (25) et (26) portant les deux demi-bulbes de chaque groupe d'extrémité,
- engage les extrémités d'assemblage de chaque demi-bulbe correspondant l'une dans l'autre pour fermer les deux demi-bulbes de chaque bulbe et constituer ainsi des bulbes d'extrémité entiers (27) et (28), **caractérisé en ce que** l'on:
- modifie le moule pour agrandir le canal de liaison (20) et constituer des cavités ou chambres périphériques (31) et (32) au niveau des jonctions entre les extrémités de la tige (2) et celle des bulbes d'extrémité, ainsi que des espaces pour la formation d'une surépaisseur périphérique au niveau de chaque extrémité,
- injecte une deuxième matière pour former la tige (2) et par extension sur et au-delà des extrémités de la tige (2) et sur les extrémités assemblées des demi-bulbes de chaque bulbe d'extrémité par la deuxième matière une surépaisseur de solidarisation formant un bourrelet ou une bague de jonction et de solidarisation (33) et (34) destinée à renforcer la jonction entre l'extrémité de la tige (2) et chaque bulbe d'extrémité (27) et (28).

2. Procédé de fabrication d'un instrument de nettoyage ou de prélèvement selon la revendication 1, **caractérisé en ce que** la liaison (24) entre les deux bulbes d'extrémité (27) et (28) est une liaison filaire.

3. Procédé de fabrication d'un instrument de nettoyage ou de prélèvement selon la revendication 1 ou 2, **caractérisé en ce que** l'on augmente la section du canal (20) avant l'injection de la deuxième matière.

4. Procédé de fabrication d'un instrument de nettoyage ou de prélèvement selon l'une quelconque des revendications 1 à 3, **caractérisé en ce que** l'on forme la tige (2) par injection d'une matière autour de la liaison de la structure de base (19) reliant les deux bulbes d'extrémité (27) et (28).

5. Procédé de fabrication d'un instrument de nettoyage ou de prélèvement selon l'une quelconque des revendications 1 à 4, **caractérisé en ce que** la première matière est un polyéthylène.

6. Procédé de fabrication d'un instrument de nettoyage ou de prélèvement selon l'une quelconque des revendications de 1 à 5, **caractérisé en ce que** la deuxième matière est un polypropylène.

7. Instrument de nettoyage ou de prélèvement obtenu par le procédé selon l'une quelconque des revendications 1 à 6, comportant une tige (2) et au moins un moyen de curetage solidaire de l'une des extrémités de la tige (2), ledit moyen de curetage étant constitué d'une pluralité de lamelles (9) individualisées, lesquelles sont reliées entre elles au niveau d'une de leurs extrémités et solidarisées par leur autre extrémité à l'extrémité de la tige (2), de manière à constituer à cette extrémité de la tige (2), une forme bombée susceptible de déformation, **caractérisé en ce qu'**au moins quelques-unes des lamelles (9) présentent en saillie sur au moins une partie de leur face extérieure délimitant la forme bombée, des brins flexibles (35).

8. Instrument de nettoyage ou de prélèvement selon la revendication 7, **caractérisé en ce que** les brins flexibles (35) sont réalisés d'une seule pièce avec les lamelles (9).

9. Instrument de nettoyage ou de prélèvement selon la revendication 7 ou 8, **caractérisé en ce que** les brins flexibles (35) présentent de préférence un diamètre inférieur à 0,3 mm.

10. Instrument de nettoyage et de prélèvement selon l'une quelconque des revendications 7 à 9, **caractérisé en ce que** les brins flexibles (35) présentent de préférence une longueur comprise entre 1,5 et 3 mm.

11. Instrument de nettoyage et de prélèvement selon l'une quelconque des revendications 7 à 10, **caractérisé en ce que** l'espacement entre les brins flexibles (35) est compris de préférence entre 1 et 3 mm.

12. Instrument de nettoyage et de prélèvement selon l'une quelconque des revendications 7 à 11, **caractérisé en ce que** la tige (2) comporte à une extrémité, un premier moyen de curetage dont les lamelles (9) présentent des brins flexibles (35) et à une autre extrémité un second moyen de curetage dont les lamelles (9) sont dépourvues de brins flexibles (35).

13. Instrument de nettoyage et de prélèvement selon l'une quelconque des revendications 7 à 11, **caractérisé en ce que** la tige (2) comporte à chacune de ses extrémités, un moyen de curetage dont les lamelles (9) présentent des brins flexibles (35).

## Patentansprüche

1. Herstellungsverfahren eines Reinigungs- oder Entnahmeinstruments in einem natürlichen oder künstlichen körperlicher Kanal eines menschlichen oder tierischen Körpers vom Typ Stifte (2), von denen jedes Ende einen flexiblen Endkopf (27) oder (28) umfasst, der aus einer Folge von Lamellen (9) gebildet ist, wobei jeder Kopf durch die Überlagerung von zwei Halbköpfen geformt ist, die untereinander durch ein Scharnier (14) oder (15) artikuliert und per Abdeckung und Immobilisierung ihrer Enden zusammengesetzt sind, durch die sie am entsprechenden Ende des Stiftes (2) verbunden sind, in dem :
- per Einspritzen eines ersten Materials in eine Form (18) eine Basisstmktur (19) gebildet wird, die zwei Endgruppen aus zwei flach offenen Halbköpfen (10, 11) oder (12, 13) aufweist, die jeweils in einem Teil der Form mit schwenkbarem Keil (25) oder (26) getragen werden,
- per Einspritzen dieses ersten Materials in einen Verbindungskanal (20) eine Verbindung (24) zwischen den zwei Endgruppen derart gebildet wird, dass die Basisstmktur (19) komplett gebildet wird, indem die zwei schwenkbaren Keile (25) und (26) umgeschlagen werden, die die zwei Halbköpfe jeder Endgruppe tragen,
- die entsprechenden Montageenden jedes Halbkopfes ineinander zum Eingreifen gebracht werden, um die zwei Halbköpfe jedes Kopfes zu schließen und somit ganze Endköpfe (27) und (28) zu bilden, **dadurch gekennzeichnet, dass**:
- die Form abgeändert wird, um den Verbindungskanal (20) zu vergrößern und Aushöhlungen oder umlaufende Kammern (31) und (32) an den Angrenzungen zwischen den Enden des Stiftes (2) und dem der Endköpfe sowie Räume für die Bildung einer umlaufenden Überdicke an jedem Ende zu bilden,
- ein zweites Material eingespritzt wird, um den Stift (2) und per Erweiterung auf den Enden des Stiftes (2) und darüber hinweg und auf den zusammengebauten Enden der Halbköpfe jedes Endkopfes durch das zweite Material eine Überdicke zur festen Befestigung zu formen, die einen Wulst oder einen Angrenzungs- und festen Befestigungsring (33) und (34) bildet, der zur Verstärkung der Angrenzung zwischen dem Ende des Stiftes (2) und jedem Endkopf (27) und (28) bestimmt ist.

2. Herstellungsverfahren eines Reinigungs- oder Entnahmeinstruments gemäß Anspruch 1, **dadurch gekennzeichnet, dass** die Verbindung (24) zwischen den zwei Endköpfen (27) und (28) eine Fadenverbindung ist.

3. Herstellungsverfahren eines Reinigungs- oder Entnahmeinstruments gemäß Anspruch 1 oder 2, **dadurch gekennzeichnet, dass** der Querschnitt des Kanals (20) vor dem Einspritzen des zweiten Materials vergrößert wird.

4. Herstellungsverfahren eines Reinigungs- oder Entnahmeinstruments gemäß irgendeinem der Ansprüche 1 bis 3, **dadurch gekennzeichnet, dass** der Stift (2) per Einspritzen eines Materials um die Verbindung der Basisstruktur (19) geformt wird, die die zwei Endköpfe (27) und (28) verbindet.

5. Herstellungsverfahren eines Reinigungs- oder Entnahmeinstruments gemäß irgendeinem der Ansprüche 1 bis 4, **dadurch gekennzeichnet, dass** das erste Material ein Polyethylen ist.

6. Herstellungsverfahren eines Reinigungs- oder Entnahmeinstruments gemäß irgendeinem der Ansprüche 1 bis 5, **dadurch gekennzeichnet, dass** das zweite Material ein Polypropylen ist.

7. Reinigungs- oder Entnahmeinstrument, das durch ein Verfahren gemäß irgendeinem der Ansprüche 1 bis 6 erhalten wird, umfassend einen Stift (2) und wenigstens ein Kuretagemittel, das fest an einem der Enden des Stiftes (2) befestigt ist, wobei das genannte Kuretagemittel aus einer Vielzahl von individualisierten Lamellen (9) gebildet ist, die untereinander an einem ihrer Enden verbunden und durch ihr anderes Ende am Ende des Stiftes (2) derart fest befestigt sind, dass an diesem Ende des Stiftes (2) eine bombierte Form gebildet wird, die sich verformen kann, **dadurch gekennzeichnet, dass** wenigstens einige der Lamellen (9) hervorstehend auf wenigstens einem Teil ihrer die bombierte Form begrenzenden Außenseite flexible Fasern (35) aufweisen.

8. Reinigungs- oder Entnahmeinstrument gemäß Anspruch 7, **dadurch gekennzeichnet, dass** die flexiblen Fasern (35) aus einem einzigen Stück mit den Lamellen (9) realisiert sind.

9. Reinigungs- oder Entnahmeinstrument gemäß Anspruch 7 oder 8, **dadurch gekennzeichnet, dass** die flexiblen Fasern (35) bevorzugt einen Durchmesser von unter 0,3 mm aufweisen.

10. Reinigungs- oder Entnahmeinstrument irgendeinem der Ansprüche 7 bis 9, **dadurch gekennzeichnet, dass** die flexiblen Fasern (35) bevorzugt eine zwischen 1,5 und 3 mm inbegriffene Länge aufweisen.

11. Reinigungs- und Entnahmeinstrument gemäß irgendeinem der Ansprüche 7 bis 10, **dadurch gekennzeichnet, dass** die Beabstandung zwischen den flexiblen Fasern (35) bevorzugt zwischen 1 und 3 mm inbegriffen ist.

12. Reinigungs- und Entnahmeinstrument gemäß irgendeinem der Ansprüche 7 bis 11, **dadurch gekennzeichnet, dass** der Stift (2) an einem Ende ein erstes Kuretagemittel trägt, dessen Lamellen (9) flexible Fasern (35) aufweisen, und an einem anderen Ende ein zweites Kuretagemittel, dessen Lamellen (9) keine flexiblen Fasern (35) aufweisen.

13. Reinigungs- und Entnahmeinstrument gemäß irgendeinem der Ansprüche 7 bis 11, **dadurch gekennzeichnet, dass** der Stift (2) an jedem seiner Enden ein Kuretagemittel umfasst, dessen Lamellen (9) flexible Fasern (35) aufweisen.

## Claims

1. A method of manufacturing a cleaning or sampling instrument in the natural or artificial bodily cavity of a human or animal body of the stem (2) type each end of which has a flexible terminating bulb (27) or (28) comprising a succession of strips (9) with each bulb being formed of the superpositioning of two half-bulbs articulating with one another by a hinge (14) or (15) and assembled by the overlapping and immobilization of their ends by which they are connected to the corresponding end of the stem (2), wherein:
- a basic structure (19) having two end groups of two half-bulbs (10, 11) or (12, 13) opened flat, each borne by a part of a swiveling tab mold (25) or (26), is formed by the injection of a first material into a mold (18),
- a link (24) between the two end groups is formed by the injection of this first material into an interconnecting channel (20), in such a way as to form the entire basic structure (19) by folding down the two swiveling tabs (25) and (26) supporting the two half-bulbs of each end group,
- the assembly ends of each of the corresponding half-bulb are engaged in each other to close the half-bulbs of each bulb thus forming the complete end bulbs (27) and (28),
**characterized in that**:
- the mold is modified to enlarge the interconnecting channel (20) and form peripheral cavities or chambers (31) and (32) at the connections between the ends of stem (2) and the end bulbs, and spaces for the forming of a peripheral extra thickness at each end,
- a second material is injected to form the stem (2) and by extension on and beyond the ends of the stem (2) and on the assembled half-bulbs ends of each end bulb by the second material a strengthening extra thickness forming an attaching and strengthening burr or ring (33) and (34) designed to reinforce the connection between the end of stem (2) and each end bulb (27) and (28).

2. A method of manufacturing a cleaning or sampling instrument according to claim 1, **characterized in that** the link (24) between the two end bulbs (27) and (28) is a filar link.

3. A method of manufacturing a cleaning or sampling instrument according to claim 1 or 2 **characterized in that** the size of the section of channel (20) is increased before the injection of the second material.

4. A method of manufacturing a cleaning or sampling instrument according to any one of claims 1 to 3, **characterized in that** the stem (2) is formed by the injection of a material around the link of basic structure (19) connecting the two end bulbs (27) and (28).

5. A method of manufacturing a cleaning or sampling instrument according to any one of claims 1 to 4, **characterized in that** the first material is a polyethylene.

6. A method of manufacturing a cleaning or sampling instrument according to any one of claims 1 to 5, **characterized in that** the second material is a polypropylene.

7. A cleaning or sampling instrument obtained by the method relating to any one of claims 1 to 6, including a stem (2) and at least one curetting means integral with one of the ends of stem (2), said curetting means consisting of a multitude of individualized strips (9) which are connected together at one of their ends and made integral by their other end with the end of stem (2), in order to form at this end of stem (2) a domed shape liable to deform, **characterized in that** at least some of the strips (9), have, protruding over at least one part of their outer surface delimiting the domed shape, flexible lengths (35).

8. A cleaning or sampling instrument according to claim 7, **characterized in that** the flexible lengths (35) are formed in one part with the strips (9).

9. A cleaning or sampling instrument according to claim 7 or 8, **characterized in that** the flexible lengths (35) preferably have a diameter of less than 0.3 mm.

10. A cleaning or sampling instrument according to any one of claims 7 to 9, **characterized in that** the flexible lengths (35) preferably have a length comprised between 1.5 and 3 mm.

11. A cleaning and sampling instrument according to any one of claims 7 to 10, **characterized in that** the interval between the flexible lengths (35) is preferably comprised between 1 and 3 mm.

12. A cleaning and sampling instrument according to any one of claims 7 to 11, **characterized in that** stem (2), at one end, has a first curetting means the strips (9) of which have flexible lengths (35) and, at the other end, a second curetting means the strips (9) of which are devoid of flexible lengths (35).

13. A cleaning and sampling instrument according to any one of claims 7 to 11, **characterized in that** the stem (2), at either end, has a curetting means the strips (9) of which have flexible lengths (35).
